# EUROPEAN PATENT SPECIFICATION

(11) **EP 1 550 465 B1**
(45) Date of publication and mention of the grant of the patent: **22.05.2013**
(21) Application number: 02765427.6
(22) Date of filing: 05.09.2002
(51) Int. Cl.: A61L 2/06, A61B 1/12, A61L 2/07, A61L 2/24, A61B 1/00

(54) **STERILIZER AND STERILIZING METHOD**
STERILISATOR UND STERILISATIONSVERFAHREN
STERILISATEUR ET PROCEDE DE STERILISATION

(43) Date of publication of application: 06.07.2005
(73) Proprietor: Olympus Corporation, Tokyo 151-0072 (JP)
(72) Inventor: NOGUCHI, Toshiaki, Tachikawa-shi, Tokyo 190-0002 (JP); MORIYAMA, Hiroki, Akishima-shi, Tokyo 196-0032 (JP); OSHIMA, Ryu, Hachioji-shi, Tokyo 193-0832 (JP); AMANO, Shoichi, Tokyo 193-0825 (JP); ISHIBIKI, Kota, Hachioji-shi, Tokyo 192-0916 (JP); MORISHITA, Koji, Mitaka-shi, Tokyo 181-0004 (JP); FUTATSUGI, Yasuyuki, Hachioji-shi, Tokyo 193-0825 (JP); HASEGAWA, Hitoshi, Kohoku-ku, Yokohama-shi, Kanagawa 222-002 (JP); NAKAGAWA, Mikihiko, Hachioji-shi, Tokyo 192-0024 (JP); YOSHIMOTO, Yosuke, Hachioji-shi, Tokyo 192-0912 (JP); TASHIRO, Yoshio, Hachioji-shi, Tokyo 192-0916 (JP)
(74) Representative: von Hellfeld, Axel
(86) International application number: PCT/JP2002/009063
(87) International publication number: WO 2004/022109

(56) References cited:
- JP-A- 5 285 103
- JP-A- 5 337 082
- JP-A- 5 337 171
- JP-A- 2002 253 477
- JP-A- 2002 263 172
- JP-A- 2002 306 412
- US-A- 4 862 872

## Description

### Technical Field

The present invention relates to a sterilizing apparatus and a sterilizing method for sterilizing a to-be-sterilized object such as an endoscope.

### Background Art

After an endoscope is used in an inspection, the endoscope is cleaned by a cleaning apparatus to remove contamination on the endoscope. Subsequently, the endoscope is subjected to high-pressure steam in an autoclave apparatus, and the endoscope is sterilized to prevent infection. In this way, the cleaning and sterilization of the endoscope are independently performed by the different apparatuses.

If contamination on the endoscope is not removed, steam for sterilization does not sufficiently come in contact with that part of the surface of the endoscope, where contamination is present, in a process of sterilizing the endoscope by means of a sterilizing apparatus. Consequently, the effect of sterilization cannot fully be obtained. Besides, if there is a hole or a flaw in the endoscope, electronic components that are built in the endoscope would be damaged by exposure to high-pressure steam. To prevent this, a leak check for checking the presence of a hole or a flaw needs to be executed before the endoscope is sterilized by the sterilizing apparatus.

The endoscope should be sterilized by the sterilizing apparatus after the endoscope has exactly undergone the cleaning process and leak check. However, the user has to check whether the endoscope has undergone the cleaning process and leak check.

In addition, the user manually inputs conditions for the sterilization process to the sterilizing apparatus. However, as will be described later, the work of exactly inputting information associated with the conditions of individual endoscopes is complex and time-consuming due to peculiar problems of endoscopes. The load on the user side is great, and the user is required to have relatively high-level expertise.

Endoscopes have various structures depending on their types. There are endoscopes with complex channel structures, endoscopes with large channel lengths, and endoscopes with very thin channels. Such endoscopes require a considerable length of time for a sterilizing process until steam for sterilization reaches all parts of the channels.

In the case of an endoscope having no channels or a short, thick endoscope with a simple channel structure, steam can reach all parts of channels in a short time period and a process time may be short. In addition, in the case of an endoscope with resistance to high temperatures and high pressures, the sterilization process time for the endoscope can be reduced by raising the set values of the temperature and pressure for the sterilizing process.

On the other hand, in the high-pressure steam sterilization process, the endoscope that is to be sterilized is placed in a process chamber and exposed to high-temperature steam. Immediately after sterilization, the body of the endoscope has a high temperature due to remaining heat. Electronic components that are built in the body of the endoscope, in particular, an imaging component such as a CCD (Charge-Coupled Device), are affected by the heat at the time of sterilization and their functions are degraded. Noise, etc. may be included in an image, and the quality of the image may deteriorate. In order to use the endoscope successively, it is necessary to wait until the endoscope is completely cooled.

In a step of disinfecting/sterilizing the endoscope using an autoclave apparatus, a high-pressure/high-humidity atmosphere is created in the process chamber. In addition, in order to facilitate flow of steam into the process chamber, the process chamber is evacuated prior to starting the process.

In the case where the endoscope is disinfected/sterilized by the autoclave apparatus, the user can change the settings relating to the process to be executed. For example, an autoclave apparatus, which is disclosed in Jpn. Pat. Appln. KOKAI Publication No. 5-285103, enables the user to change the settings relating to the process to be executed. In the case where the settings of the process to be executed are freely changeable, the process time can advantageously be decreased. However, if proper settings are not made, the endoscope may be damaged in the sterilizing process and the durability of the endoscope may be degraded.

The above-mentioned prior art document relating to the autoclave apparatus describes that the durability of the endoscope is secured by adjusting the pressure in the chamber and the pressure in the endoscope. This document, however, does not describe the relation between the variations of the process and the durability of the endoscope. Nor does the document mention the control of the dry state of the inside gas in the endoscope. The dry state of the inside of the endoscope, which has been autoclaved, is closely related to the durability of the endoscope. If much moisture remains in the endoscope, the durability of the endoscope tends to decrease. However, the addition of an excessive drying step leads to an increase in process time and is disadvantageous when a quick process is needed. Besides, if proper conditions for sterilization can be set depending on the condition in which the endoscope was used or the state of contamination, degradation in the endoscope can be prevented.

Document US 4,862,872 A discloses to store in a memory section of an endoscope patient data which reflects the usage of the endoscope with regard to the patient in the past. Cleaning/sterilizing processes of the endoscope are controlled in dependence on the stored patient data.

### Disclosure of Invention

The present invention has been made in consideration of the above problems. An object of the invention is to provide a sterilizing apparatus that can reduce a load on a user, and can exactly and quickly execute a sterilizing process under a proper sterilization process condition.

Another object of the invention is to provide a sterilizing apparatus that can prevent degradation in an endoscope due to variations in a sterilizing process using an autoclave.

The present invention provides a sterilizing apparatus according to claim 1.

According to the invention, cleaning information and sterilization information is stored in a to-be-sterilized object such as an endoscope. Based on the information, the process is automatically confirmed and corrected. Therefore, the invention can provide a sterilizing apparatus that can reduce a load on a user, and can exactly and quickly execute a process.

The invention also provides a sterilizing method according to claim 11.

In the invention, information relating to the type, history, etc. of a to-be-sterilized object is stored. Based on the information, the process condition is automatically set and confirmed, and correction is made to the to-be-sterilized object. According to the invention, it is possible to prevent degradation in an endoscope due to variations in a sterilizing process.

### Brief Description of Drawings

FIG. 1 is a side view that schematically shows the entirety of an endoscope according to a first embodiment;
FIG. 2 is a perspective view of an endoscope cleaning apparatus according to the first embodiment;
FIG. 3 is a perspective view of an endoscope sterilizing apparatus according to the first embodiment;
FIG. 4 is a perspective view of the entirety of an endoscopy system according to the first embodiment;
FIG. 5 is a block diagram of the endoscopy system according to the first embodiment;
FIG. 6 is a circuit diagram of an RFID unit and an RFID tag of the endoscopy system according to the first embodiment;
FIG. 7 shows the structure of an endoscope apparatus according to a second embodiment;
FIG. 8 is a perspective view that shows the structure of an autoclave apparatus according to the second embodiment;
FIG. 9 is a time chart that illustrates an autoclave process using the autoclave apparatus shown in FIG. 8;
FIG. 10 is a flow chart that illustrates the autoclave process using the autoclave apparatus shown in FIG. 8;
FIG. 11 shows the structure of an autoclave apparatus according to a third embodiment;
FIG. 12 is a flow chart that illustrates an autoclave process using the autoclave apparatus shown in FIG. 11; and
FIG. 13 schematically shows the structure of an autoclave apparatus according to a fourth embodiment.

### Best Mode for Carrying Out the Invention

### (First Embodiment)

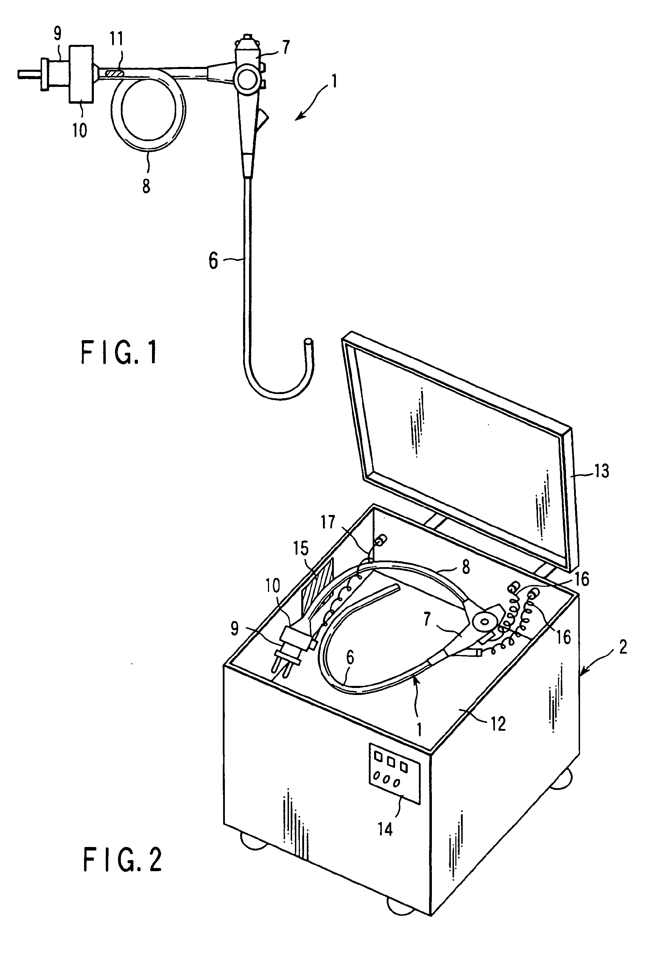

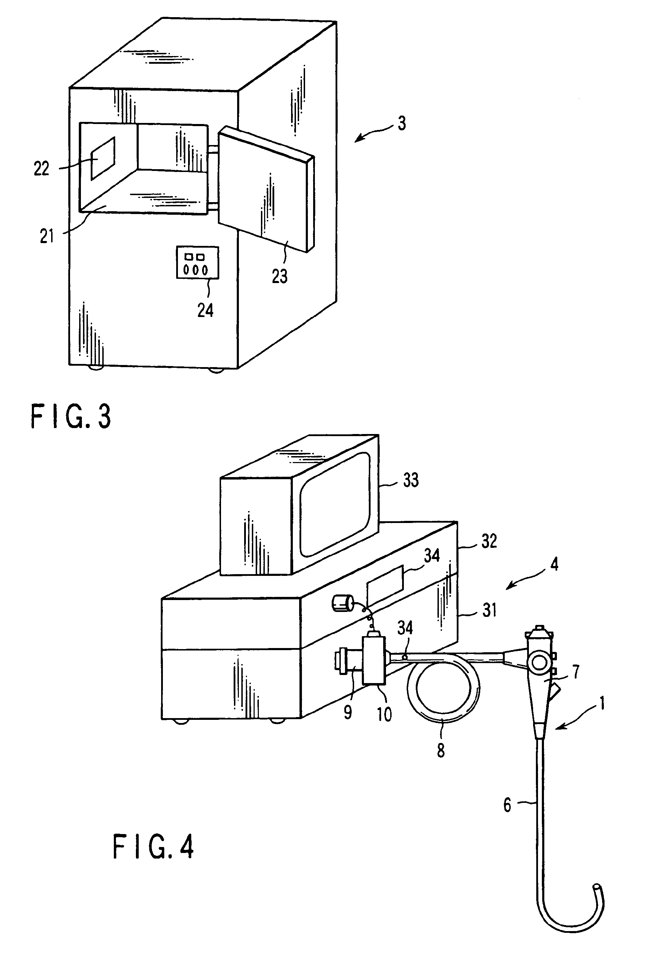

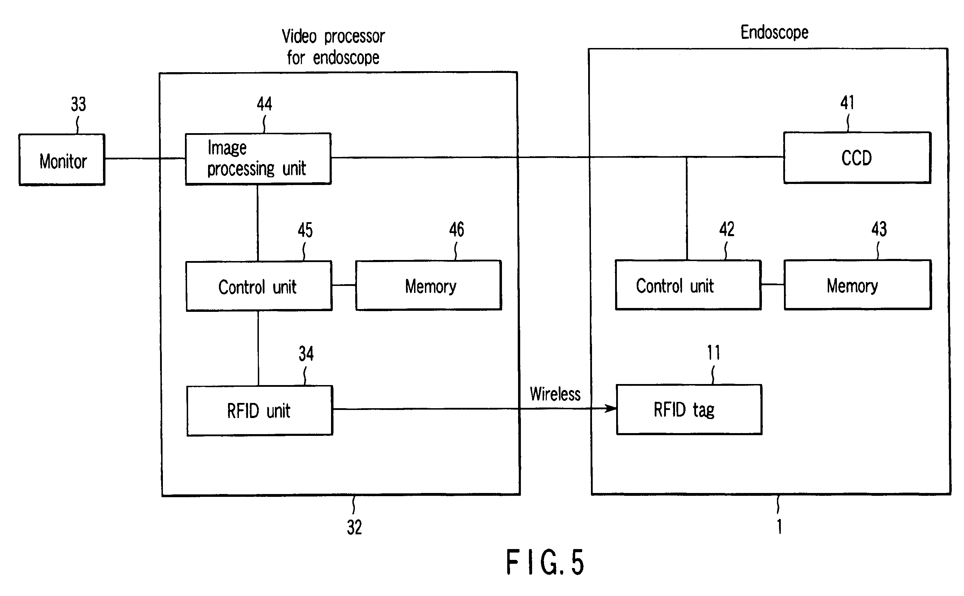

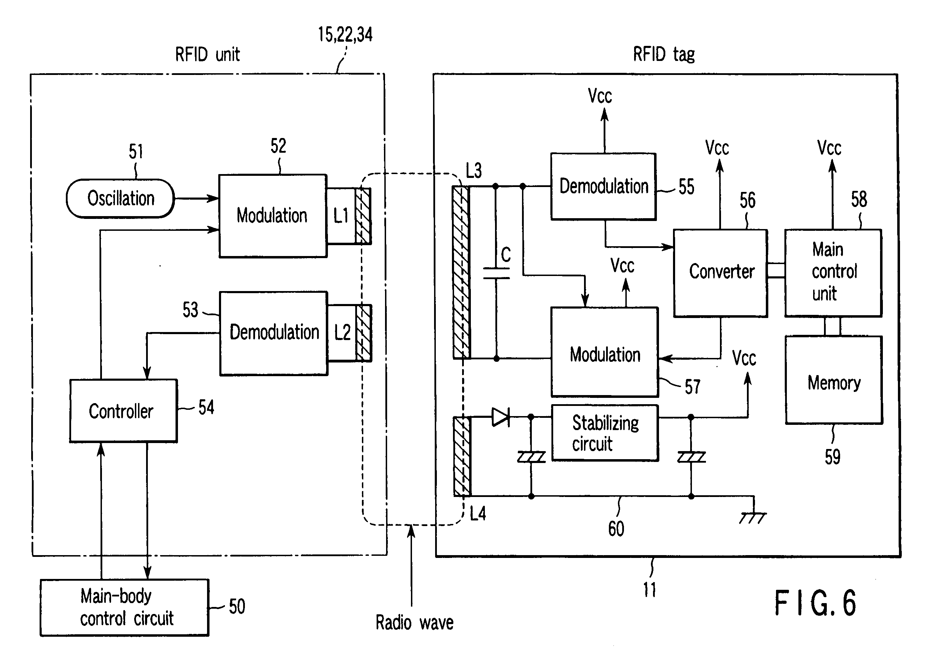

An endoscope cleaning apparatus according to a first embodiment of the invention will now be described with reference to FIG. 1 to FIG. 6. FIG. 1 schematically shows an endoscope 1. FIG. 2 shows an endoscope cleaning apparatus 2 for cleaning the endoscope 1. FIG. 3 shows an endoscope sterilizing apparatus 3 for sterilizing the endoscope 1. FIG. 4 schematically shows an endoscopy system 4. FIG. 5 is a block diagram of the endoscopy system 4, and FIG. 6 is a circuit diagram of an RFID unit and an RFID tag.

The endoscope 1 includes an insertion section 6, an operation section 7 and a light guide cable 8. A connector 9 is provided at an extension end of the light guide cable 8. A communication connector section 10 for electrical connection to a peripheral device (not shown) is provided in the vicinity of the connector 9. The communication connector section 10 is provided with a connector portion for leakage testing (not shown). The communication connector 10 is equipped with a waterproof cap (not shown) that covers an exposed part of an electrical connection portion of the communication connector 10. When the endoscope 1 is cleaned and sterilized, the waterproof cap (not shown) prevents entrance of liquid such as a detergent or a disinfectant and protects the endoscope 1.

An RFID tag 11 is provided at a position on the light guide cable 8 near the connector 9. The RFID tag 11 executes data transmission/reception in a non-electrical-contact method with an RFID unit (to be described later) that is provided on another apparatus such as the endoscope video processor, endoscope cleaning apparatus 2 or endoscope sterilizing apparatus 3. The RFID tag 11 includes a CPU (Central Processing Unit), a transmission/reception circuit, an antenna and a memory.

The basic principle of the non-electrical-contact method is a communication system using RFID (radio frequency identification) that uses radio waves. Examples of the RFID include "electromagnetic coupling method", "capacitive coupling method", "electromagnetic induction method", "microwave method" and "optical communication method." In general, RFID is applicable not only to data transmission/reception but also to power transmission/reception.

The memory (characteristic information recording section) of the RFID tag 11 stores characteristic information that includes history information as to whether the cleaning of the endoscope 1 that is to be sterilized is completed, as to when the cleaning process was executed, as to the result of leakage testing, as to whether sterilization is completed, and as to when sterilization was performed. In addition to the history information, the characteristic information includes information relating to the model name, i.e., type, of the endoscope 1, the product serial number of the endoscope 1, the time when an endoscopic inspection was conducted, and the case of treatment for which the endoscope 1 was used.

An RFID system (wireless automatic recognition system) is constituted by the RFID tag 11 of the endoscope 1 and an RFID unit that is included in each of the endoscope video processor 32, endoscope cleaning apparatus 2 and endoscope sterilizing apparatus 3, which are described later. Information transmission/reception and power supply are executed through the communication using the RFID system. The RFID system adopts a wireless communication method using high-frequency waves, and not a wired method using electrical contact. Thus, both the RFID tag 11 and RFID unit can be sealed with members of a resin, etc., and both the RFID tag 11 and RFID unit have complete waterproof structures.

The endoscope cleaning apparatus 2 shown in FIG. 2 comprises a cleaning bath 12 that accommodates the endoscope 1 as a medical instrument to be cleaned, a top cover 13, and a control panel 14 that sets sterilization process conditions such as a process step program.

The RFID unit 15 is provided on a part of the cleaning bath 12. The RFID unit 15 constitutes an information reading unit that executes communication with the RFID tag 11 of the endoscope 1 that is accommodated in the cleaning bath 12, and reads record information in the RFID tag 11 that stores information such as the model name, product serial number, history, etc. of the endoscope 1.

The endoscope cleaning apparatus 2 includes cleaning process condition setting means (step condition calculating section) that sets process conditions for cleaning the endoscope 1 accommodated in the cleaning bath 12, on the basis of the information of the endoscope 1 that is read out of the endoscope 1 accommodated in the cleaning bath 12. The RFID unit 15 is composed of a transmission/reception circuit, an antenna, etc., and is controlled by a control unit that is built in the endoscope cleaning apparatus 2.

When the endoscope 1 is cleaned by the endoscope cleaning apparatus 2, the endoscope 1 is set in the cleaning bath 12, as shown in FIG. 2. Endoscope channel cleaning tubes 16 are connected to the endoscope 1 in order to clean the channels in the endoscope 1. A detergent is fed into the channels of the endoscope 1 through the cleaning tubes 16 from the endoscope cleaning apparatus 2 side. Thereby, the inside of each channel of the endoscope can be cleaned.

A leak test tube 17 is connected to the connector portion for leakage testing in order to perform a leak test step for confirming that the outer member of the endoscope 1 has no hole or flaw. When the leak test tube 17 is connected to the endoscope 1, the leak test tube 17 communicates with the inside of the endoscope 1. Then, a leak test step is started. Air is fed into the endoscope 1 through the leak test tube 17 from the endoscope cleaning apparatus 2. An air feed pressure at this time is measured by a leak sensor. If there is no hole or flaw in the endoscope 1, the air that is fed into the endoscope 1 does not leak, and a predetermined pressure value is indicated. If leakage occurs, the pressure value decreases and a leak portion of the endoscope 1 is detected. In this manner, it can be confirmed whether there is a hole or flaw in the endoscope 1.

As is shown in FIG. 3, the endoscope sterilizing apparatus 3 includes a chamber (containing section) 21 for accommodating the endoscope 1 to be sterilized. An RFID unit 22 is provided on a part of the chamber 21. Like the above-described RFID unit 22 of the endoscope cleaning apparatus 2, the RFID unit 22 is composed of a transmission/reception circuit, an antenna, etc. The RFID unit 22 is controlled by a control unit in the endoscope sterilizing apparatus 3. The RFID unit 22 executes wireless communication with the RFID tag 11 of the endoscope 1 and constitutes communication means for reading record information of the RFID tag 11 that stores information such as the model name, product serial number, history, etc. of the endoscope 1 accommodated in the chamber 21. In addition, the RFID unit 22 constitutes sterilization process condition setting means for setting process conditions for sterilizing the endoscope I accommodated in the chamber 21, on the basis of the characteristic information of the endoscope 1 that is read out of the endoscope 1 accommodated in the chamber 21 of the endoscope sterilizing apparatus 3.

A cover 23 is provided on the inlet of the chamber 21. A control panel 24 is provided on the front surface of the endoscope sterilizing apparatus 3. Sterilization process conditions can be set through the control panel 24.

FIG. 4 schematically shows the endoscopy system 4. The endoscopy system 4 includes a light source device 31, a video processor 32 and a monitor 33. The video processor 32 is provided with an RFID unit 34. The RFID unit 34 is controlled by a control unit that is provided in the video processor 32. The RFID unit 34 executes wireless communication with the RFID tag 11 of the endoscope 1.

FIG. 5 is a block diagram of the endoscopy system 4. The endoscope 1 includes a CCD 41, a control unit 42 and a memory 43. In addition, the endoscope 1 includes the above-described RFID tag 11. The video processor 32 for the endoscope includes an image processing unit 44, a control unit 45 and a memory 46. In addition, the video processor 32 includes the above-described RFID unit 34.

At a time of endoscopy, a video signal that is obtained by the CCD 41 of the endoscope 1 is sent to the image processing unit 44 of the video processor 32. The video signal is converted to an image signal by the image processing unit 44. The image signal is sent to the monitor 33, and the monitor 33 that serves as indication means displays an image. Wireless communication is executed by the RFID system between the endoscope 1 and video processor 32.

Wireless communication is executed between the RFID tag 11 of endoscope 1 and the RFID unit 34. Prior to endoscopy, the cleaning information and sterilization information of the endoscope 1 and the characteristic information of the model number, etc. of the endoscope 1 are read in from the RFID tag 11. After the endoscopy, the information relating to the content of endoscopy, etc. is written in the RFID tag 11.

FIG. 6 shows circuit configurations of the RFID unit 15, 22, 34 and the RFID tag 11. The RFID units 15, 22 and 34 are provided on the cleaning apparatus 2, sterilizing apparatus 3 and video processor 32, respectively.

Each of the RFID unit 15, 22 and 34 comprises a transmission unit, a receiving unit, and a controller 54. The transmission unit includes a modulation circuit 52 having an oscillation circuit 51 for transmission, and a transmission coil L1. The receiving unit that includes a receiving coil L2 and a demodulation circuit 53. The controller 54 controls the transmission/reception by these units. The controller 54 is connected to a main-body control circuit 50 that is provided in the apparatus main body.

The RFID tag 11 of the endoscope 1 comprises a coil L3 for signal transmission/reception, a capacitor C for oscillation, a demodulation circuit 55, a converter 56, a modulator 57, a main control unit 58 for controlling the respective components, and a memory 59. The RFID tag 11 includes a power supply circuit 60 that smoothes/rectifies a signal, which is received by a coil L4, and produces a stabilized drive power. The respective components are driven by the power produced by the power supply circuit 60.

Next, the operation of the present embodiment is described. To begin with, when the endoscope 1 is to be subjected to a cleaning process, the endoscope 1 is set in the endoscope cleaning apparatus 2, and a leak test process is executed to confirm whether there is a hole or a flaw in the endoscope 1. If a flaw in the endoscope 1 is detected in the test, an alarm is issued to the user and the user is prompted to immediately repair the endoscope 1. It is better to also perform a channel clogging detection step for confirming whether the endoscope channel is clogged.

The cleaning process for the endoscope 1 is started after confirming that there is no flaw in the endoscope 1 and no problem would arise even if the endoscope 1 is immersed in washing water.

After the cleaning process for the endoscope 1 is finished, the endoscope cleaning apparatus 2 activates the RFID unit 15 and executes communication with the RFID tag 11 of the endoscope 1. Cleaning information as to when the cleaning of the endoscope 1 was performed and as to the result of the leak test step is written in the RFID tag 11 of the endoscope 1. Specifically, in the RFID unit 15, the coil L1 is supplied with a radio-frequency wireless signal that is digitally modulated according to a write command, which is transferred as a serial signal from the controller 54 under the control of the main-body control circuit 50 of the endoscope cleaning apparatus 2. In the RFID tag 11 in the endoscope 1, the coil L3 receives the wireless signal that is sent from the coil L1 of the RFID unit 15. A signal that is induced by the wireless signal is amplified and demodulated into the original digital signal in the demodulation circuit 55. Based on the digital serial signal information, the main control unit 58 writes information in the memory 59. In this way, communication is executed between the RFID tag 11 and RFID unit 15, and information is stored in the memory 59 in the RFID tag 11 of the endoscope 1.

After the endoscope 1 is cleaned, the endoscope 1 is set in the chamber 21 of the sterilizing apparatus 3, and the sterilizing process for the endoscope 1 is executed. When the sterilizing process operation is started, the sterilizing apparatus 3 communicates with the endoscope 1 and confirms the cleaning information and the model number of the endoscope 1, which is stored in the RFID tag 11 of the endoscope 1. Specifically, in the RFID unit 22, the coil L1 is supplied with a radio-frequency wireless signal that is digitally modulated according to a read command, which is transferred as a serial signal from the controller 54 under the control of the main-body control circuit 50 of the sterilizing apparatus 3. In the RFID tag 11 in the endoscope 1, a signal that is induced in the coil L3 is amplified and demodulated into the original digital signal in the demodulation circuit 55. Based on the digital serial signal information, the main control unit 58 of the RFID tag 11 reads in data from the memory 59, and converts the data to a serial signal in sync with a non-modulated signal sent from the coil L1. According to this signal, the modulation circuit 57 executes a control as to whether a tank circuit of the coil L3 and capacitor C is set in a resonant state.

The controller 54 in the RFID unit 22 detects, through the coil L2, whether the tank circuit in the RFID tag 11 resonates. Thereby, communication of information is executed. In this way, communication is executed between the RFID tag 11 and RFID unit 22, and information is read out of the memory 59 in the RFID tag 11 of the endoscope 1.

If the obtained information indicates that the endoscope 1 has not been cleaned or the result of the leak test step shows the possibility of a flaw in the endoscope 1, the sterilizing apparatus 3 does not start the sterilizing process operation and issues an alarm to the user to confirm these matters.

If it is determined that the sterilizing process is executable on the basis of the information stored in the memory 59 in the RFID tag 11 of the endoscope 1, the sterilizing process operation is started. The contents of the sterilization process conditions are automatically set on the basis of the information of the model number, etc. of the endoscope 1. For example, in the case of a long endoscope for colonoscopy or an endoscope with a very thin channel such as a forceps-raising wire guide, a long time is needed until steam for sterilization reaches all parts of the channel. Thus, the process time is set to be long. On the other hand, in the case of an endoscope with a simple channel structure or an endoscope with a short channel, the process time is set to be short. In the case of an endoscope of the type that is resistant to higher sterilization steam temperatures or higher pressures than normal levels, the sterilization steam temperature and pressure are increased and the process time is set to be short. The sterilizing process conditions can also be set in accordance with the information relating to the history of use of the endoscope 1 or the history of cleaning/sterilization. Thus, the sterilizing process is executed on the basis of the content of the sterilizing process that is properly set according to the endoscope 1 to be sterilized.

If the sterilizing process for the endoscope 1 is completed, the sterilizing apparatus 3 executes communication with the endoscope 1 once again and stores sterilization information, which relates to the time and kind of the sterilizing process, into the memory 59 in the RFID tag 11 of the endoscope 1.

Subsequently, when an inspection is to be conducted using the sterilized endoscope 1, the endoscope 1 is connected to the light source device 31 and video processor 32, as shown in FIG. 4. The video processor 32 communicates with the connected endoscope 1 and confirms the sterilization information. If it is confirmed that the endoscope 1 is a sterilized one, the sterilization information as to what kind of sterilizing process was executed and when it was done is displayed on the monitor 33, and the user is prompted to confirm the information. If it is determined that the endoscope 1 is not a sterilized one, the monitor 33 displays a corresponding alarm message to the user.

The video processor 32 confirms the sterilizing process content and estimates the temperature of the CCD 41 of the endoscope 1. The video processor 32 prestores data relating to a temperature decrease of the CCD 41 of the endoscope 1 in the case where the endoscope 1 is sterilized and then left in the natural state. By communication with the endoscope 1, the video processor 32 recognizes an elapsed time from the completion of the sterilizing process and thus calculates the temperature of the CCD 41. When an endoscopic image that is taken by the CCD 41 is subjected to image processing, a filtration constant for removing noise due to the heat of the CCD 41 is calculated on the basis of the temperature of the CCD 41. Thereby, correction of the endoscopic image is automatically executed. Thus, the endoscope 1 can continuously be used without waiting for a long time until the CCD 41 in the endoscope 1 is completely cooled down.

As has been described above, in the present embodiment, the video processor, cleaning apparatus and sterilizing apparatus for the endoscope are configured to be able to write, by communication, the cleaning information and sterilization information in the memory in the endoscope, and to read out information from the memory. Based on the information, the process conditions are automatically set or corrected. Thereby, the load on a user is reduced, and the exact and quick process can be executed.

When the endoscope is subjected to the cleaning process in the cleaning apparatus, the cleaning apparatus communicates with the endoscope and receives information relating to the endoscope. In addition, the cleaning apparatus stores information relating to the cleaning in the memory means of the endoscope 1. The information relating to the cleaning, in this context, refers to, for instance, information as to when the cleaning process was executed, information as to what kind of cleaning process was executed, information relating to the result of a leak test step for confirming whether leakage occurs in the endoscope, and information relating to the result of a channel clogging detection step for confirming whether the endoscope channel is clogged.

When the endoscope is subjected to the sterilizing process in the sterilizing apparatus, the endoscope sterilizing apparatus communicates with the endoscope and acquires information relating to the cleaning and information relating to the endoscope itself, which are stored in the endoscope. Based on the information, the endoscope sterilizing apparatus confirms whether the endoscope 1 has exactly been cleaned. In addition, the endoscope sterilizing apparatus confirms whether the endoscope is free from leakage or channel clogging. If it is not possible to confirm such information, an alarm may be issued to the user to prevent the sterilizing process from being started.

On the other hand, the conditions for the sterilizing process are automatically set in accordance with the type of the endoscope. When the sterilizing process is completed, the endoscope sterilizing apparatus 3 executes communication with the endoscope 1 once again and stores the information relating to sterilization in the endoscope. The information relating to sterilization, in this context, refers to, for instance, information as to what kind of sterilizing process was executed and when the sterilizing process as executed.

When endoscopy is conducted using the sterilized endoscope, the endoscope image processing apparatus communicates with the endoscope and acquires information relating to the sterilization. Based on the information as to what kind of sterilizing process was executed and when the sterilizing process as executed, the endoscope image processing apparatus estimates the temperature in the inside of the endoscope and executes, for example, a noise-reduction image process in accordance with the temperature characteristics of the CCD.

### (Second Embodiment)

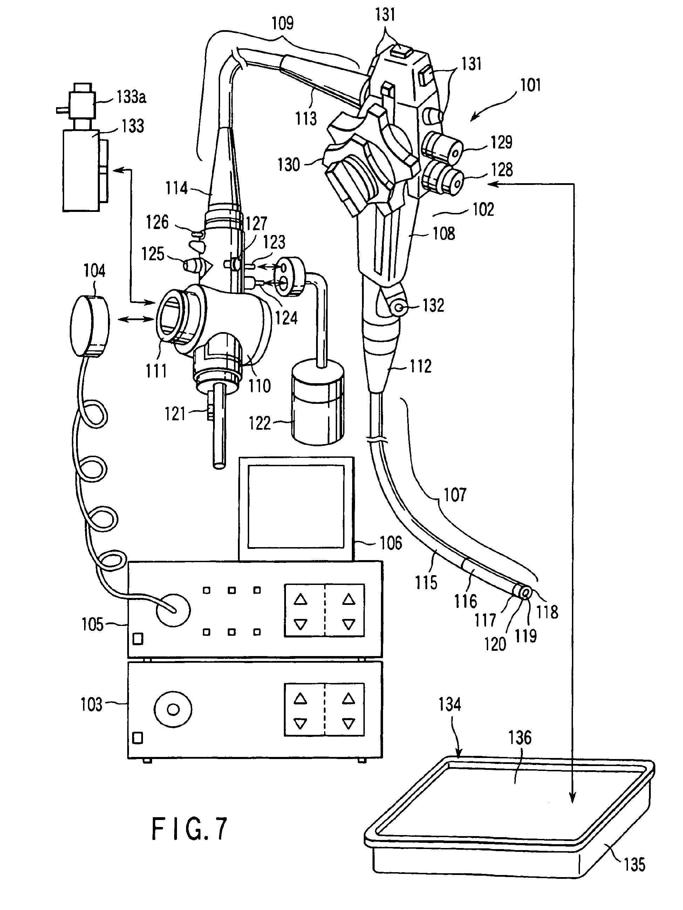

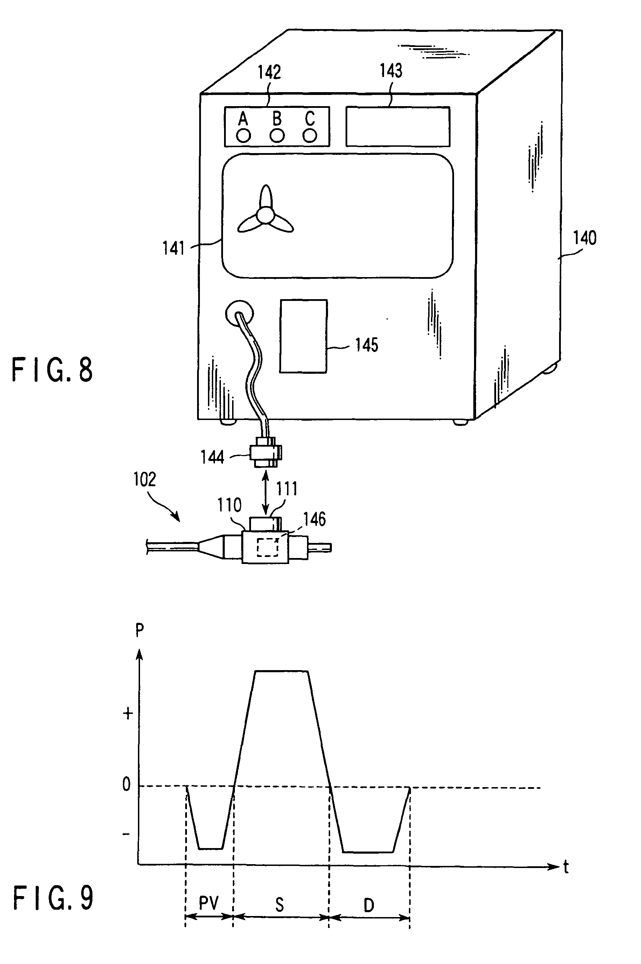

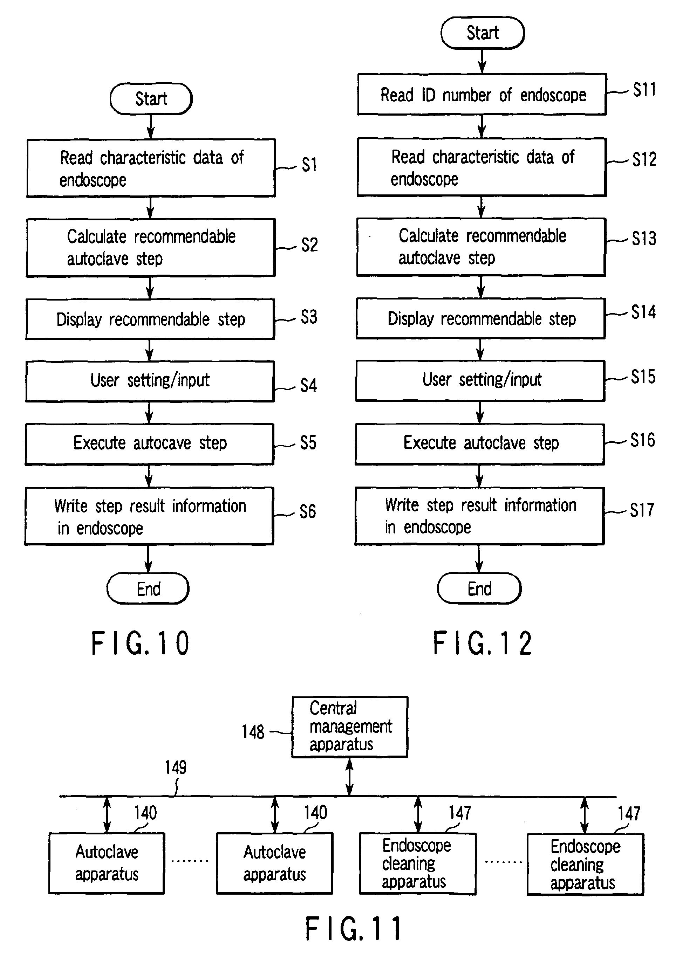

An autoclave apparatus according to a second embodiment of the invention will now be described with reference to FIG. 7 to FIG. 10. FIG. 7 is an explanatory view that schematically shows the structure of an endoscope. FIG. 8 is an explanatory view that schematically shows the structure of an autoclave apparatus for sterilizing the endoscope shown in FIG. 7. FIG. 9 is a time chart of an autoclave sterilization process using the autoclave apparatus shown in FIG. 8. FIG. 10 is a flow chart that illustrates the autoclave sterilization process by the autoclave apparatus shown in FIG. 8.

As is shown in FIG. 7, an endoscope apparatus 101 includes an endoscope 102 with imaging means; a light source device 103 that is detachably connected to the endoscope 102 and supplies illumination light to a light guide (not shown) that is provided on the endoscope 102; a video processor 105 that is connected to the endoscope 102 via a signal cable 104, controls the imaging means of the endoscope 102, and processes a video signal obtained by the imaging means; and a monitor 106 that displays a video image corresponding to a subject image, which is output from the video processor 105.

The endoscope 102 is formed such that the endoscope 102 can be subjected to a cleaning process after it is used for observation or treatment, and the endoscope 102, which has been subjected to the cleaning process, can subsequently be subjected to a sterilizing process using high-pressure steam. The endoscope 102 includes a flexible thin, long insertion section 107; an operation section 108 that is connected to a proximal end side of the insertion section 107; a flexible connection cord 109 that extends from a side part of the operation section 108; and a connection section 110 that is provided at an extension end of the connection cord 109 and is detachably connected to the light source device 103. A side part of the connector section 110 is provided with an electric connector section 111 that is detachably connectable to the signal cable 104 connected to the video processor 105. The electric connector section 111 is provided with an air passage portion (not shown) for communication between the inside of the endoscope 102 and the outside.

A connection part between the insertion section 107 and the operation section 108 is provided with an insertion-section-side breakage-preventing member 112 that has an elastic member for preventing sharp bending of the connection part. Similarly, a connection part between the operation section 108 and connection cord 109 is provided with an operation-section-side breakage-preventing member 113. In addition, a connector-section-side breakage-preventing member 114 is provided at a connection part between the connection cord 109 and connection section 110.

The insertion section 107 comprises a soft, flexible tube portion 115; a bendable portion 116 that is provided at a distal end side of the flexible tube portion 115 and can be bent by the operation of the operation section 108; and a distal end portion 117 that is provided at a distal end of the flexible tube portion 115 and is provided with an observation optical system and an illumination optical system (not shown).

The distal end portion 117 of the insertion section 107 is provided with an air-feeding/water-feeding nozzle 118 for feeding a detergent or gas toward an optical member that is provided on the outer surface of the observation optical system (not shown) by an air-feeding operation and a water-feeding operation; a suction portion 119 that is a distal-end-side opening of an instrument channel (not shown) for insertion of an instrument provided in the insertion section 107 or for suction of liquid in a body cavity; and a liquid-feeding port 120 that is open to an object of observation and feeds liquid.

As is shown in FIG. 7, the connector section 110 is provided with a gas-feeding mouthpiece 121 that is detachably connected to a gas supply source (not shown) that is built in the light source device 103; a water-feeding tank pressurizing mouthpiece 123 and a liquid supply mouthpiece 124, which are detachably connected to a water-feeding tank 122 that is a liquid supply source; a suction mouthpiece 125 that is connected to a suction source (not shown) for effecting suction from the suction port 119; and an injection mouthpiece 126 that is connected to water-feeding means (not shown) for feeding water from the liquid-feeding port 120. The connector section 110 is provided with a ground terminal mouthpiece 127 for feeding leak current back to a high-frequency treatment device when high-frequency leak current occurs in the endoscope in the case of conducting high-frequency treatment, etc.

The operation section 108 is provided with an air-feed/water-feed operation button 128 for executing an air-feed operation and a water-feed operation; a suction operation button 129 for executing a suction operation; a bending operation knob 130 for executing a bending operation of the bendable portion; a plurality of remote switches 131 for remote-controlling the video processor 105; and an instrument insertion port 132 that is an opening communicating with the instrument channel.

A watertight cap 133 with a pressure adjusting valve is detachably connectable to the electric connector section 111 of the endoscope 102. The watertight cap 133 is equipped with a pressure adjusting valve 133a.

When the endoscope 102 is sterilized with high-pressure steam, a sterilization case 134 for storing the endoscope 102 is used. The sterilization case 134, as shown in FIG. 7, comprises a tray 135 and a cover member 136. The tray 135 and cover member 136 are provided with a plurality of air passage holes (not shown). Steam can pass through the air passage holes.

The connector section 110 is provided with a restriction member (not shown) that corresponds to the shape of the endoscope 102. The restriction member is formed such that the respective parts of the endoscope 102 are placed at predetermined positions. The restriction member is provided with an insertion section restriction portion (not shown) in which the elongated flexible section 107 is stored.

Typical conditions for high-pressure steam sterilization are U.S. standards ANSI/AAMI ST37-1992, which are endorsed by American National Standards Institute and issued by Association for the Advancement of Medical Instrumentation. According to these standards, in the case of the pre-vacuum type, the time for a sterilization step is four minutes at 132°C. In the case of the gravity type, the time for a sterilization step is 10 minutes at 132°C. Thus, the temperature condition for the sterilization step with high-pressure steam is generally set in the range of about 115°C to 138°C, although the temperatures vary depending on the type of the high-pressure steam sterilizing apparatus and the time of the sterilization step. In some types of sterilizing apparatuses, the temperature can be set at about 142°C.

The time condition varies depending on the temperature condition for the sterilization step. In general, the time is set between about 3 minutes and 60 minutes. In some types of sterilizing apparatuses, the time can be set at about 100 minutes. In this step, the pressure in the sterilization chamber is generally set at about +0.2 MPa, relative to the atmospheric pressure.

The high-pressure steam sterilization step for the general pre-vacuum type includes a pre-vacuum step for setting the sterilization chamber, where the to-be-sterilized apparatus is accommodated, in a vacuum state prior to sterilization, and a subsequent sterilization step for feeding high-pressure, high-temperature steam into the sterilization chamber and executing sterilization.

The pre-vacuum step is a step for making the steam reach minute portions of the to-be-sterilized apparatus in the subsequent sterilization step. The pressure in the sterilization chamber is reduced so that high-pressure, high-temperature steam may reach every part of the to-be-sterilized apparatus. The pressure in the sterilization chamber in the pre-vacuum step is generally set at about -0.07 MPa to -0.09 MPa, relative to the atmospheric pressure.

The sterilization step is followed by a drying step for setting the inside of the sterilization chamber in a reduced-pressure state once again, thereby to dry the sterilized apparatus. In the drying step, the sterilization chamber is decompressed to remove steam from the sterilization chamber. This facilitates the drying of the to-be-sterilized apparatus in the sterilization chamber. The pressure in the drying sterilization chamber in the drying step is generally set at about -0.07 MPa to -0.09 MPa, relative to the atmospheric pressure.

When the endoscope 102 is sterilized with high-pressure steam, the sterilization is performed in the state in which the watertight cap 133 with the pressure adjusting valve is attached to the electric connector section 111. In this state, the pressure adjusting valve 133a of the watertight cap 133 is closed, and the passage hole is closed by the watertight cap 133. Thus, the inside of the endoscope 102 is watertightly sealed from the outside.

When the sterilization is executed by the sterilizing process including the pre-vacuum step, the pressure in the sterilization chamber is reduced in the pre-vacuum step, and such a pressure difference occurs that the outside pressure becomes lower than the inside pressure of the endoscope 102. At this time, the pressure adjusting valve 133a is opened and the inside and outside of the endoscope 102 communicate via the air passage hole. This prevents the occurrence of a large pressure difference between the inside of the endoscope 102 and the inside of the sterilization chamber. Hence, when the endoscope is subjected to the sterilizing process, no damage occurs due to the pressure difference between the inside and outside.

If the sterilization chamber is pressurized in the sterilization step and such a pressure difference occurs that the pressure in the outside of the endoscope 102 becomes higher than the pressure in the inside of the endoscope 102, the pressure adjusting valve 133a is closed. Thus, high-pressure, high-temperature steam does not positively enter the endoscope 102 via the watertight cap 133 and air passage hole. However, high-temperature, high-pressure steam gradually enters the endoscope 102 through, for example, the outer sheath of the flexible tube formed of high-polymer material or an O-ring formed of fluoro-rubber, silicone rubber, etc., which is a sealing means provided at a connection part of the outer member of the endoscope 102.

A pressure acting from outside toward inside, which is a sum of the reduced pressure in the pre-vacuum step and the applied pressure in the sterilization step, is applied to the outer member of the endoscope 102.

In the case of the process in which the sterilization step is followed by a pressure-reducing step, if the pressure-reducing step is completed, the sterilization chamber is pressurized. As a result, such a pressure difference occurs that the pressure in the outside of the endoscope 102 becomes higher than the pressure in the inside of the endoscope 102, and the pressure adjusting valve 133a is closed.

If all steps for high-pressure steam sterilization are completed, a pressure acting inward from outside, which corresponds to the reduced pressure in the pressure-reducing step, is applied to the outer member of the endoscope 102. If the watertight cap 133 is removed from the electric connector section 111, the inside and outside of the endoscope 102 communicate through the air passage hole and the inside pressure of the endoscope 102 changes to the atmospheric pressure. As a result, the pressure load on the outer member of the endoscope 102 is eliminated.

On the other hand, a chamber 141 for accommodating the endoscope 102 and executing an autoclave process is formed in an autoclave apparatus 140. In addition, the autoclave apparatus 140 includes a water tank, a steam generating device and a suction pump, which are not shown.

As is shown in FIG. 8, the autoclave apparatus 140 includes a process step selection section 142, a process step display section 143, an endoscope data reading device 144, and an endoscope data display section 145. The autoclave apparatus 140 also includes a write control section 125 that executes a control to write an executed treatment process condition, as history information, in the memory section of the endoscope 102; a step condition calculation section 126 that calculates a sterilization process condition relating to the step; and a control section 128. The control section 128 controls the sterilization step of the autoclave apparatus 140 in accordance with the sterilization process condition that is set by the step condition calculation section 126.

The process step selection section 142 is provided with a plurality of operation buttons of A, B and C. Needless to say, the number of buttons may be four or more, or two.

The endoscope data reading device 144 shown in FIG. 8 is detachably connectable to the electric connector section 111 of the endoscope 102. The endoscope data reading device 144 is connected to the autoclave apparatus 140 by means of a soft communication cord 144a.

The connector section 110 incorporates a memory unit 146 that stores endoscope data. If the endoscope data reading device 144 is connected to the electric connector section 111, the autoclave apparatus 140 is enabled to read the data stored in the memory unit 146. The endoscope data reading device 144 can write executed process content, as history information, in the memory unit 146 of the to-be-processed endoscope 102. In the present embodiment, the endoscope data reading device 144 functions both as an apparatus for reading data and as an apparatus for writing data.

Data may be written to and read from the memory unit 146 of the autoclave apparatus 140 by means of data transmission/reception using a non-electric-contact communication method, without using the apparatus 144 with the soft cord 144a. RFID (radio frequency identification) that makes use of radio waves may be adopted as the non-electric-contact method. Examples of the RFID include "electromagnetic coupling method", "capacitive coupling method", "electromagnetic induction method", "microwave method" and "optical communication method." This method is applicable not only to data transmission/reception but also to power transmission/reception.

Next, the operation of the autoclave apparatus of the present embodiment with the above-described structure is described. FIG. 9 illustrates, in a simplified fashion, the autoclave sterilization step that is executed by the autoclave apparatus 140. In the graph of FIG. 9, the ordinate indicates a pressure state (0 = atmospheric pressure) and the abscissa indicates the passing of time.

Generally speaking, a single autoclave sterilization process comprises a pre-vacuum step (PV step) for setting the inside of the chamber 141 in a negative-pressure state, a high-pressure steam sterilization step (S step) and a negative-pressure drying step (D step).

In the PV step, a negative pressure is built, following which steam is injected. Thereby, steam can be sufficiently fed to thin channels in the endoscope 102. In the negative-pressure state, the inside of the endoscope 102 tries to have a higher pressure than the inside of the chamber 141. However, the pressure in the endoscope 102 is kept at a level close to the pressure in the chamber 141 by the function of the pressure adjusting valve 33a of the watertight cap 133. Thus, such a problem, as breakage of the bendable portion 116, does not occur.

In the S step, at the time of maximum pressure, the temperature reaches a sterilization temperature of, e.g. 135°C. In the S step, a small amount of steam enters the endoscope 102 via the soft flexible tube portion 115 or soft sealing portions at some locations (the outside pressure (i.e. pressure in the chamber 141) is much higher than the inside pressure of the endoscope 102).

In the D step, the inside of the chamber 141 is dried with hot air while the inside of the chamber 141 is kept at a negative pressure. By setting the inside of the chamber 141 at a negative pressure, the pressure adjusting valve 133a functions and most of the steam, which has entered the endoscope 102 in the S step, can be removed.

FIG. 10 illustrates process steps relating to the handling of the data stored in the memory unit 146 of the endoscope 102. The flow of the process, as well as these steps, is described.

In step S1 of "read characteristic data of the endoscope", the watertight cap 133 of the cleaned endoscope 102 is once removed, and the endoscope data reading device 144 is connected to the electric connector section 111. Then, the autoclave apparatus 140 reads the information that is stored in the memory unit 146 of the endoscope 102.

The information includes history information relating to, e.g. the PV step time and negative-pressure level, the S step time and temperature, and D step time and negative-pressure level in the previously executed autoclave processes. The history information, which relates to, e.g. previous ten steps, may be displayed on the endoscope data display section 145. In addition, the information that is stored in the memory unit 146 may include information such as the model type of the endoscope 102, other than the history information.

In step S2 of "calculate a recommendable autoclave step", the autoclave apparatus 140 calculates, based on the read data, a recommendable step as an autoclave step that is next executed. For example, if it is determined that the previous D step time was shorter than a proper time, a step in which the D step time is relatively long is recommended.

In step S3 of "display a recommendable step", the content of the recommendable step is displayed on the process step display section 143, thus informing the user.

The endoscope 102 is placed in the chamber 141. Prior to this, the watertight cap 133 is attached to the electric connector section 111.

In the next step S4 of "user setting/input", the buttons on the process step selection section 142 designate three autoclave step patterns A, B and C.

For example, pattern A is most suitable to the endoscope 102, but the D step time is long. Pattern B is usable when a relatively quick process is required, and thus the D step time is relatively short. Pattern C relates to a process of autoclaving an instrument such as an accessory, a cleaning instrument or a treatment instrument, which has a simpler structure than the endoscope 102. In pattern C, both the PV step time and D step time are very short. That is, all the steps of the autoclave process can be completed in a very short time period. Pattern C is applicable to the endoscope 102 when a quick process is particularly required. However, since the D step time is very short, the application of pattern C to the endoscope 102 leads to accumulation of moisture in the endoscope 102, degrading the durability of the endoscope 102.

In step S4 of "user setting/input", if pattern A was previously applied to the endoscope 102 in succession, the process step display section 143 displays a message to the effect that any one of patterns A to C is applicable. If a quick process is needed, the user can select pattern C.

If pattern C was previously executed, the autoclave apparatus 140 that reads the associated data causes the display section 143 to display a message to the effect that pattern A is recommendable.

The user thus presses the button A. Depending on cases, the autoclave apparatus 140 may be configured to become inoperable if the user presses buttons other than the button A.

If the user selects a certain autoclave step, the next step S5 of "execute an autoclave step" is performed and the autoclave operation is started.

If the step of "execute an autoclave step" is completed, step S6 of "write step result information in the endoscope" is executed. The endoscope 102 is taken out of the chamber 141, and the watertight cap 133 is removed from the electric connector section 111. The endoscope data reading device 144 is connected to the electric connector section 111 once again. The executed process content is written as history information in the memory unit 146 of the endoscope 102 that is the object of processing.

As has been described, at least a part of the data relating to the autoclave process, which is executed each time, is managed for each endoscope. Based on the data, an autoclave process that is next executed is determined. Thereby, the durability of the endoscope 102 is secured. In particular, the D step is an important step for minimizing the moisture in the endoscope 102. If the user desires a quick process and the D step time becomes inadequate, the durability of the endoscope 102 is seriously affected.

For example, in the case of a patient with Creutzfeldt-Jakob disease, it is necessary to make the temperature of the S step higher and the time of the S step longer than the levels in the ordinary autoclave condition.

Although the number of such patients is small, this very severe autoclave process condition may lead to damage to the endoscope if the same endoscope is subjected to the autoclave process many times. Thus, this embodiment may adopt such management that the number of times and the frequency of execution of the process with the above-mentioned special condition are recorded, and the same endoscope is prevented from being subjected to the process many times.

The patterns A, B and C of the autoclave process may be associated with displays that recommend applicable products such as "flexible endoscope", "rigid endoscope" and "forceps". Further, buttons, which are indicative of "flexible endoscope", "rigid endoscope", "forceps" and "linen", may be provided. If the objects that are placed in the chamber 41 are a forceps and linen, the user may press the buttons indicative of "forceps" and "linen". Then, the autoclave apparatus 40 executes calculations and determines a process that is suitable for autoclaving the two objects, and automatically operates.

As has been described above, according to the present embodiment, the user can select the autoclave process in accordance with the need at each time, and the durability of the endoscope can be secured.

### (Third Embodiment)

An autoclave apparatus according to a third embodiment is described referring to FIG. 11 and FIG. 12. The present embodiment is substantially the same as the second embodiment. Different points are mainly described. The common structural parts are denoted by like reference numerals, and a detailed description is omitted. FIG. 11 is an explanatory view that shows the structure of the autoclave apparatus that subjects the endoscope to autoclave-sterilization. FIG. 12 is a flow chart illustrating the flow of the autoclave process using the autoclave apparatus shown in FIG. 11.

As is shown in FIG. 11, in the present embodiment, an autoclave apparatus 140 and an endoscope cleaning apparatus 147 are connected to a central management apparatus 148 by a signal line 149. Specifically, the autoclave apparatus 140 itself does not execute data management and processing. The data management and processing are executed by the central management apparatus 148. As is shown in FIG. 11, a plurality of autoclave apparatuses 140 and a plurality of endoscope cleaning apparatuses 147 may be provided. In this case, too, the data management/processing is executed by the common central management apparatus 148. In the other structural aspects, the present embodiment is the same as the second embodiment.

Next, the operation of the present embodiment is described. As is illustrated in FIG. 12, in step S11, the endoscope data reading device 144 is connected to the electric connector section 111 before the endoscope 102 is placed in the chamber 141. Thus, the central management apparatus 148 reads out the ID number of the endoscope from the memory unit 146.

The central management apparatus 148 stores past records relating to the ID number. In step S12, the central management apparatus 148 reads out the data that relates to, for instance, the PV step time and negative-pressure level, the S step time and temperature and the D step time and negative-pressure level in the previously executed autoclave step. Then, the central management apparatus 48 executes the process of "calculate a recommendable autoclave step" in step S13.

In step S14, information relating to a recommendable process is sent to the autoclave apparatus 140 and the process step display section 143 is caused to display the information, thereby notifying the user.

Subsequently, in step S15, the user sets (inputs) the process to be executed. In the second embodiment, one of the buttons on the process step selection section 142 is selected. The process step selection section 142 may be composed of a touch panel such that the user can freely input, e.g. the D step time in units of a minute. If the input content differs from the recommendable process content that is displayed on the process step display section 143, the central management apparatus 148 may execute determination and may effect alarm display on the process step display section 143. In addition, the central management apparatus 148 may control the autoclave apparatus 140 so as to prohibit the operation of the autoclave apparatus 140.

In step S16, the autoclave sterilization is executed. If the sterilization is completed, the information relating to the executed process is sent to the central management apparatus 148 in step S17 and is stored in the memory unit as the information associated with the ID number.

Information of the temperature sensor and pressure sensor that are provided in the chamber 141 may be recorded as management information as to whether the autoclave apparatus 140 has normally operated in fact. If abnormality occurs, the central management apparatus 148 executes a control to suspend the process and to effect display on the process step display section 143 to prompt the user to perform a check.

In addition to checking whether the autoclave apparatus 140 normally operates, it is possible to check abnormality of the endoscope 102 by sensing an instantaneous change in pressure of the chamber 141 when the pressure adjusting value 133a malfunctions and the bendable portion 116 is broken. The chamber 141 may be provided with a sound sensor to sense burst sound.

The endoscope 102 is cleaned by the endoscope cleaning apparatus 147 before executing autoclave sterilization. The endoscope cleaning apparatus 147 may also be provided with the endoscope data reading device 144 and the central management apparatus 148 may manage the information as to what kind of cleaning was done.

The central management apparatus 148 may be connected to the video processor 105. If the user connects the signal cable 104 to the electric connector section 111 at the time of an inspection, it becomes possible to quickly and exactly understand whether the endoscope has normally been cleaned/sterilized.

As has been described above, the common central management apparatus 148 can manage/control the plural endoscopes 102, endoscope cleaning apparatuses 147 and autoclave apparatuses 140. Therefore, the structure of the memory unit 146 can be made simpler, and the computers (information processing units, control units) in the autoclave apparatus 140 and endoscope cleaning apparatus 147 can be dispensed with. In particular, in a large-scale hospital, it becomes possible to comprehensively secure the exact information management and the durability of endoscopes at a minimum cost.

According to the present embodiment, in addition to the advantageous effect of the second embodiment, an efficient system can be provided in the case where a plurality of autoclave apparatuses 140 are employed.

### (Fourth Embodiment)

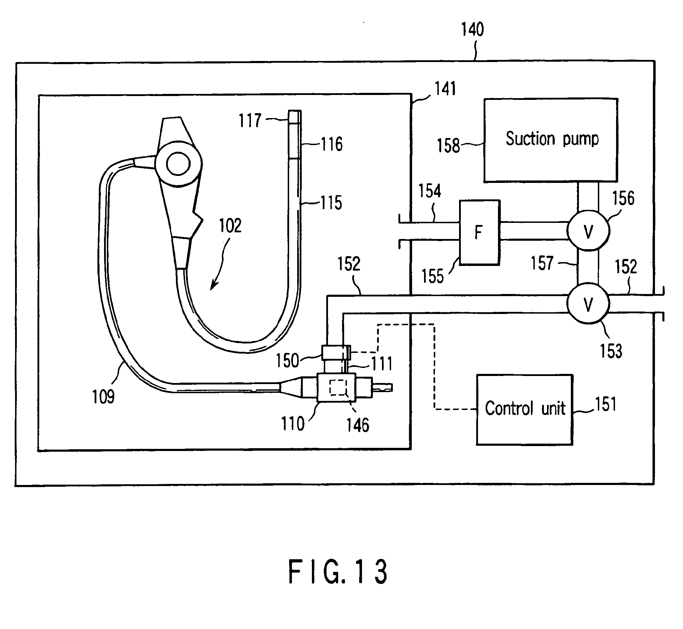

An autoclave apparatus according to a fourth embodiment is described referring to FIG. 13. The present embodiment is substantially the same as the third embodiment. Only different points are described. The common structural parts are denoted by like reference numerals, and a detailed description is omitted. FIG. 13 is an explanatory view that schematically shows the structure of an autoclave apparatus for autoclaving the endoscope 102 in the state in which the endoscope 102 is placed in the chamber 141.

In the present embodiment, an endoscope connection section 150, which is provided on the autoclave apparatus 140 and is substituted for the above-described watertight cap 133, is connected to the electric connector section 111. Thereby, a control unit 151 of the autoclave apparatus 140 is rendered communicable with the memory unit 146. The inside of the endoscope 102 communicates with a conduit 152. The conduit 152 communicates with the outside of the autoclave apparatus 140 via a control valve 153. A conduit 154 communicates with the chamber 141 and reaches a control valve 156 via a filter 155 that blocks passage of micro-organisms.

The control valve 153 communicates with the control valve 156 via a conduit 157. The control unit 151 controls the control valve 153, control valve 156, suction pump 158 and the steam generating device (not shown). In the other structural aspects, the present embodiment is the same as the second embodiment.

Next, the operation of this embodiment is described. The conduit connection section 150 is connected to the watertight cap 111. Thereby, like the second embodiment, information is read out of the memory unit 146 and the control unit 151 executes processing.

A variation in pressure in the chamber 141 of the autoclave process is controlled in the following manner. For example, when the chamber 141 is set at a negative pressure by the suction pump 158, the control valve 153 equalizes the pressures in the endoscope 102 and to the pressure in the chamber 141. (That is, the inside of the endoscope 102 is also set at a negative pressure by the suction pump 158.)

If the autoclave process is completed, the control valve 153 is controlled to eliminate moisture in the endoscope 102 as quickly as possible. To begin with, the inside of the endoscope 102 is sucked by the suction pump 158 and set at a negative pressure level. A suction device for sucking an inside gas in the endoscope 102 is constituted. Then, air is let into the endoscope 102 from the outside of the autoclave apparatus 140. An injection device, which injects a gas different from the gas in the endoscope into the endoscope 102 after the suction, is thus constituted.

After the inside of the chamber 141 is completely dried, gas in the chamber 141 is drawn. Using the same means described above, ventilation may be achieved. To be more specific, after the inside of the endoscope 102 is set at a negative pressure level by the suction pump 158, gas in the chamber 141 may be fed into the endoscope 102 from the endoscope connection section 150 via the control valve 156 and control valve 153 and the gas in the endoscope 102 may be replaced with the air in the chamber 141. Outside air is not necessarily in a high-level dry state. In this way, ventilation in a high-level dry state can be executed by feeding the gas in the chamber 141, which is in the high-level dry state, into the endoscope 102.

In the above-described scheme, if the temperature of the gas, which is to be fed into the endoscope 102 from the endoscope connection section 150, is lower than the temperature of the gas in the endoscope 102, quick cooling can be executed and the preparation time for endoscopy can be decreased. For example, a cooling device may be provided at a given position on the conduit 152, and cool gas may be fed into the endoscope 102 to more quickly cool the endoscope 102.

As has been described above, ventilation in the endoscope 102 can be executed using the suction pump 158 that is employed to control the pressure in the chamber 141. Therefore, there is no need to provide another pump for ventilation in the endoscope 102, and the cost is reduced.

The suction pump 158 may be provided anywhere along the flow path of the conduit 152, control valve 153, conduit 157, control valve 156 and conduit 154. Using the suction pump 158, air may be fed from the outside of the autoclave apparatus 140 into the chamber 141 via the filter 155, thereby cooling the endoscope 102. In this case, it is preferable that the cooling step be executed after the drying step for drying the inside of the chamber 141. This can reduce the cooling time, without condensation.

According the present embodiment, in addition to the advantageous effect of the second embodiment, the inside of the endoscope can easily be ventilated, and the durability can be secured.

In the above-described embodiments, the autoclave apparatus 140 may integrally be provided with the function of cleaning the endoscope 102. Thereby, the characteristic information relating to the cleaning/sterilization of the endoscope 102 can be managed with a single setting.

## Claims

1. A sterilizing apparatus (140) that executes a sterilizing step of storing an endoscope (102) which includes an information writable and readably memory section (146), in a chamber (141), and sterilizing the endoscope (102) with high-temperature, and high-pressure steam, and a drying step of drying the endoscope (102) to which moisture is added by the steam, the sterilizing apparatus (140) comprising:
a reading section (144) that reads from the memory section (146) of the endoscope (102), prestored information that includes a process condition of at least one of the sterilizing step and drying step, and a type of the endoscope (102);
a step condition calculating section that calculates at least one process condition that is to be currently executed, which relates to at least one of the sterilizing step and the drying step, on the basis of the information that is read by the reading section (144);
a control section (151) that controls a process operation of at least one of the sterilizing step and the drying step, which relates to the process condition, in accordance with the process condition that is calculated by the step condition calculating section; and
**characterized by**:
a means that detects the process condition of said at least one of the sterilizing step and the drying step, and that generates a signal which reflects the process condition; and
a write section that receives the signal, and that writes the process condition of said at least one of the sterilizing step and the drying step according to the signal, as history information, in the memory section (146) of the endoscope (102).

2. The sterilizing apparatus (140) according to claim 1, wherein the step condition calculating section calculates a plurality of different process conditions that are to be currently executed, and which further comprises an indicating section (143) that indicates said plurality of different process conditions that are calculated by the step condition calculating section; and
a selection section (142) for selecting one process condition that is to be executed for the endoscope (102), from the plurality of different indicated process conditions that are indicated by the indication section (143).

3. The sterilizing apparatus (140) according to claim2, wherein the step condition calculating section calculates a plurality of different process conditions including at least different step times, respectively.

4. The sterilizing apparatus (140) according to any one of claims 1 to 3, further comprising a suction device (158) that is connected to the endoscope (102) in the chamber (141) and sucks an internal gas of the endoscope (102), and an injection device that injects another gas different from the gas in the endoscope (102) into the endoscope (102) after the suction.

5. The sterilizing apparatus (140) according to claim 4, wherein the suction device is the same as means for executing suction of the chamber (141).

6. The sterilizing apparatus (140) according to claim4 or 5, wherein said another gas includes dry gas in the chamber (141).

7. The sterilizing apparatus (140) according to any one of claims 1 to 6, wherein the reading section (144) reads previous history information written in the memory section (146), if the history information is stored In the memory, and the step condition calculating section calculates said at least one process condition on the basis of the previous history information in addition to the information that is read by the reading section (144).

8. The sterilizing apparatus (140) according to any one of claims 1 to 7, wherein said prestored information includes both process conditions of the sterilizing step and drying step.

9. The sterilizing apparatus (140) according to any one of claims 1 to 8, wherein the reading section (144) includes a communication device that receives by radio the information from the memory section (146) of the endoscope (102).

10. The sterilizing apparatus (140) according to any one of claims 1 to 8, wherein the reading section (144) includes a communication device that includes a communication cord, which is connectable to the memory section (146) of the endoscope (102), and receives the information from the information storing section through the communication cord.

11. A sterilizing method, comprising:
a process execution step of executing a sterilizing step of sterilizing an endoscope (102) by applying heat and high-pressure steam, and a drying step of drying the endoscope (102) that is sterilized with the steam;
a reading step that reads from a memory section (146) of the endoscope, prestored information that includes a process condition of at least one of the sterilizing step and drying step, and a type of the endoscope (102);
a calculating step that calculates at least one process condition that is to be currently executed, which relates to at least one of the sterilizing step and the drying step, on the basis of the information that is read by the reading step; and
a controlling step that controls a process operation of at least one of the sterilizing step and the drying step, which relates to the process condition, in accordance with the process condition that is calculated in the calculating step;
**characterized by**:
a deteding step that detects the process condition of said at least one of the sterilizing step and the drying step; and
a writing step that writes the detected process condition of said at least one of the sterilizing step and the drying step as history information in the memory section (146) of the endoscope (102).

12. The method according to claim 11, wherein the calculating step calculates a plurality of different process conditions that are to be currently executed,
further comprising an indication step that indicates said plurality of different process conditions that are calculated by the calculating step; and
a selection step for selecting one process condition that is to be executed for the endoscope (102), from the plurality of different indicative process conditions that are indicated by the indication step.

13. The method according to claim 12, wherein the calculating step calculates a plurality of different process conditions including at least different step times, respectively.

14. The method according to any one of claims 11 to 13, further comprising a suction step which sucks internal gas of the endoscope (102) and an injection step that injects another gas different from the gas in the endoscope (102) into the endoscope after the suction step.

15. The method according to any of claims 11 to 14, wherein the reading step reads previous history information written in the memory section (146), if the history information is stored in the memory, and wherein the calculating step calculates said at least one process condition on the basis of the previous history information in addition to the information that is read by the reading step.

## Patentansprüche

1. Sterilisationsvorrichtung (140), die einen Sterilisationsschritt des Lagerns eines eine beschreibbare und lesbare Informationsspeichereinheit (146) aufweisenden Endoskops (102) in einer Kammer (141) und des Sterilisierens des Endoskops (102) mit unter hohem Druck stehenden Hochtemperatur-Dampf, und einen Trocknungsschritt des Trocknens des Endoskops (102), dem durch den Dampf Feuchtigkeit zugeführt wird, ausführt, wobei die Sterilisationsvorrichtung (140) aufweist:
eine Leseeinheit (144), die aus der Speichereinheit (146) des Endoskops (102) vorab gespeicherte Informationen, die eine Prozessbedingung des Sterilisationsschritts und/oder des Trocknungsschritts beinhalten, und die Art des Endoskops (102) ausliest;
eine Schrittbedingung-Berechnungseinheit, die basierend auf den von der Leseeinheit (144) ausgelesenen Informationen wenigstens eine gegenwärtig auszuführende Prozessbedingung berechnet, die sich auf den Sterilisationsschritt und/oder den Trocknungsschritt bezieht;
eine Steuereinheit (151), die gemäß der von der Schrittbedingung-Berechnungseinheit berechneten Prozessbedingung einen Prozessvorgang des Sterilisationsschritts und/oder des Trocknungsschritts, der sich auf die Prozessbedingung bezieht, steuert; und
**gekennzeichnet durch**:
eine Einrichtung, die die Prozessbedingung des Sterilisationsschritts und/oder des Trocknungsschritts erfasst und ein die Prozessbedingung wiedergebendes Signal erzeugt; und
eine Schreibeinheit, die das Signal empfängt und gemäß dem Signal die Prozessbedingung des Sterilisationsschritts und/oder des Trocknungsschritts als History-Informationen in die Speichereinheit (146) des Endoskops (102) schreibt.

2. Sterilisationsvorrichtung (140) nach Anspruch 1, wobei die Schrittbedingung-Berechnungseinheit eine Mehrzahl verschiedener, gegenwärtig auszuführender Prozessbedingungen berechnet, und die Sterilisationsvorrichtung (140) ferner einen Anzeigebereich (143), der die Mehrzahl verschiedener Prozessbedingungen, die von der Schrittbedingung-Berechnungseinheit berechnet werden, anzeigt; und
eine Auswahleinheit (142) zum Auswählen einer für das Endoskop (102) auszuführenden Prozessbedingung aus der Mehrzahl von verschiedenen angezeigten Prozessbedingungen aufweist, die von dem Anzeigebereich (143) angezeigt werden.

3. Sterilisationsvorrichtung (140) nach Anspruch 2, wobei die Schrittbedingung-Berechnungseinheit jeweils eine Mehrzahl von verschiedenen Prozessbedingungen, die zumindest verschiedene Schrittzeiten einschließen, berechnet.

4. Sterilisationsvorrichtung (140) nach einem der Ansprüche 1 bis 3, die ferner aufweist: eine Saugvorrichtung (158), die mit dem sich in der Kammer (141) befindenden Endoskop (102) verbunden ist und ein sich im Inneren des Endoskops (102) befindendes Gas ansaugt, und eine Einblasvorrichtung, die nach dem Saugvorgang ein anderes Gas, das sich von dem in dem Endoskop (102) befindenden Gas unterscheidet, in das Endoskop (102) einbringt.

5. Sterilisationsvorrichtung (140) nach Anspruch 4, wobei die Saugvorrichtung mit einer Einrichtung zum Durchführen eines Saugvorgangs bezüglich der Kammer (141) identisch ist.

6. Sterilisationsvorrichtung (140) nach einem der Ansprüche 4 oder 5, wobei das andere Gas trockenes Gas in der Kammer (141) einschließt.

7. Sterilisationsvorrichtung (140) nach einem der Ansprüche 1 bis 6, wobei die Leseeinheit (144) frühere, in die Speichereinheit (146) geschriebene History-Informationen ausliest, wenn die History-Informationen in dem Speicher abgespeichert sind, und die Schrittbedingung-Berechnungseinheit die wenigstens eine Prozessbedingung basierend auf den früheren History-Informationen zusätzlich zu den von der Leseeinheit (144) ausgelesenen Informationen berechnet.

8. Sterilisationsvorrichtung (140) nach einem der Ansprüche 1 bis 7, wobei die vorab gespeicherten Informationen sowohl Prozessbedingungen des Sterilisationsschritts als auch solche des Trocknungsschritts beinhalten.

9. Sterilisationsvorrichtung (140) nach einem der Ansprüche 1 bis 8, wobei die Leseeinheit (144) eine Kommunikationsvorrichtung aufweist, die über Funk die Informationen von der Speichereinheit (146) des Endoskops (102) empfängt.

10. Sterilisationsvorrichtung (140) nach einem der Ansprüche 1 bis 8, wobei die Leseeinheit (144) eine Kommunikationsvorrichtung aufweist, die eine an die Speichereinheit (146) des Endoskops (102) anschließbare Kommunikationsleitung aufweist, und die Informationen von dem Informationspeicherbereich durch die Kommunikationsleitung empfängt.

11. Sterilisationsverfahren, das die folgenden Schritte beinhaltet:
einen Prozessausführungsschritt des Ausführens eines Sterilisationsschritts des Sterilisierens eines Endoskops (102) durch Anwendung von Wärme und unter hohem Druck stehendem Dampf, und eines Trocknungsschritts des Trocknens des Endoskops (102), das mit dem Dampf sterilisiert wird;
einen Leseschritt, bei dem aus der Speichereinheit (146) des Endoskops vorab gespeicherte Informationen, die eine Prozessbedingung des Sterilisationsschritts und/oder des Trocknungsschritts beinhalten, und die Art des Endoskops (102) ausgelesen werden;
einen Berechnungsschritt, bei dem basierend auf den im Leseschritt ausgelesenen Informationen wenigstens eine gegenwärtig auszuführende Prozessbedingung berechnet wird, die sich auf den Sterilisationsschritt und/oder den Trocknungsschritt bezieht; und
einen Steuerungsschritt, bei dem gemäß der im Berechnungsschritt berechneten Prozessbedingung ein Prozessvorgang des Sterilisationsschritts und/oder des Trocknungsschritts, der sich auf die Prozessbedingung bezieht, gesteuert wird;
**gekennzeichnet durch**:
einen Erfassungsschritt, bei dem die Prozessbedingung des Sterilisationsschritts und/oder des Trocknungsschritts erfasst wird; und
einen Schreibschritt, bei dem die erfasste Prozessbedingung des Sterilisationsschritts und/oder des Trocknungsschritts als History-Informationen in die Speichereinheit (146) des Endoskops (102) geschrieben wird.

12. Verfahren nach Anspruch 11, wobei bei dem Berechnungsschritt eine Mehrzahl verschiedener, gegenwärtig auszuführender Prozessbedingungen berechnet wird,
und wobei das Verfahren ferner einen Anzeigeschritt beinhaltet, bei dem die Mehrzahl von verschiedenen Prozessbedingungen, die in dem Berechnungsschritt berechnet werden, anzeigt wird; und
einen Auswahlschritt zum Auswählen einer für das Endoskop (102) auszuführenden Prozessbedingung aus der Mehrzahl von verschiedenen angezeigten Prozessbedingungen, die in dem Anzeigeschritt angezeigt werden.

13. Verfahren nach Anspruch 12, wobei bei dem Berechnungsschritt jeweils eine Mehrzahl von verschiedenen Prozessbedingungen, die zumindest verschiedene Schrittzeiten einschließen, berechnet wird.

14. Verfahren nach einem der Ansprüche 11 bis 13, das ferner einen Saugschritt, bei dem ein sich im Inneren des Endoskops (102) befindendes Gas abgesaugt wird, und einen Einblasschritt beinhaltet, bei dem nach dem Saugvorgang ein anderes Gas, das sich von dem in dem Endoskop (102) befindenden Gas unterscheidet, in das Endoskop (102) eingebracht wird.

15. Verfahren nach einem der Ansprüche 11 bis 14, wobei bei dem Leseschritt frühere, in die Speichereinheit (146) geschriebene History-Informationen ausgelesen werden, wenn die History-Informationen in dem Speicher abgespeichert sind, und wobei bei dem Berechnungsschritt die wenigstens eine Prozessbedingung basierend auf den früheren History-Informationen zusätzlich zu den in dem Leseschritt ausgelesenen Informationen berechnet wird.

## Revendications

1. Appareil de stérilisation (140) qui exécute une étape de stérilisation consistant à stocker un endoscope (102) qui inclut une section de mémoire d'écriture et de lecture d'informations (146), dans une chambre (141), et à stériliser l'endoscope (102) sous haute température et vapeur sous haute pression, et une étape de séchage consistant à sécher l'endoscope (102) sur lequel de l'humilité est ajoutée par la vapeur, l'appareil de stérilisation (140) comprenant:
une section de lecture (144) qui lit à partir de la section de mémoire (146) de l'endoscope (102), des informations préenregistrées qui contiennent une condition de traitement d'au moins l'une parmi l'étape de stérilisation et l'étape de séchage, et un type d'endoscope (102);
une section de calcul de conditions d'étape qui calcule au moins une condition de traitement devant être actuellement exécutée, qui se rapporte à au moins l'une parmi l'étape de stérilisation et l'étape de séchage, sur la base des informations qui sont lues par la section de lecture (144);
une section de commande (151) qui commande une opération de traitement d'au moins l'une parmi l'étape de stérilisation et l'étape de séchage, qui se rapporte à la condition de traitement, conformément à la condition de traitement qui est calculée par la section de calcul de conditions d'étape; et
**caractérisé par**
un moyen qui détecte la condition de traitement de ladite au moins une parmi l'étape de stérilisation et l'étape de séchage, et qui génère un signal qui reflète la condition de traitement; et
une section d'écriture qui reçoit le signal, et qui écrit la condition de traitement de ladite au moins une parmi l'étape de stérilisation et l'étape de séchage conformément au signal, en tant qu'information historique, dans la section de mémoire (146) de l'endoscope (102).

2. Appareil de stérilisation (140) selon la revendication 1, dans lequel la section de calcul de conditions d'étape calcule une pluralité de conditions de traitement différentes devant être actuellement exécutées, et qui comprend en outre une section d'indication (143) qui indique ladite pluralité de conditions de traitement différentes qui sont calculées par la section de calcul de conditions d'étape; et
une section de sélection (142) destinée à sélectionner une condition de traitement qui doit être exécutée pour l'endoscope (102), à partir de la pluralité de conditions de traitement différentes indiquées qui sont indiquées par la section d'indication (143).

3. Appareil de stérilisation (140) selon la revendication 2, dans lequel la section de calcul de conditions d'étape calcule une pluralité de conditions de traitement différentes incluant au moins des temps d'étapes différents, respectivement.

4. Appareil de stérilisation (140) selon l'une quelconque des revendications 1 à 3, comprenant en outre un dispositif d'aspiration (158) qui est connecté à l'endoscope (102) dans la chambre (141) et aspire un gaz interne de l'endoscope (102), et un dispositif d'injection qui injecte, dans l'endoscope (102) après l'aspiration, un autre gaz différent du gaz dans l'endoscope (102).

5. Appareil de stérilisation (140) selon la revendication 4, dans lequel le dispositif d'aspiration est le même que le moyen destiné à exécuter l'aspiration de la chambre (141).

6. Appareil de stérilisation (140) selon la revendication 4 ou 5, dans lequel ledit autre gaz comprend un gaz sec dans la chambre (141).

7. Appareil de stérilisation (140) selon l'une quelconque des revendications 1 à 6, dans lequel la section de lecture (144) lit les informations historiques précédentes écrites dans la section de mémoire (146), si les informations historiques sont mémorisées dans la mémoire, et la section de calcul de conditions d'étape calcule ladite au moins une condition de traitement sur la base des informations historiques précédentes en plus des informations qui sont lues par la section de lecture (144).

8. Appareil de stérilisation (140) selon l'une quelconque des revendications 1 à 7, dans lequel lesdites informations préenregistrées contiennent à la fois les conditions de traitement de l'étape de stérilisation et de l'étape de séchage.

9. Appareil de stérilisation (140) selon l'une quelconque des revendications 1 à 8, dans lequel la section de lecture (144) comprend un dispositif de communication qui reçoit par radio les informations provenant de la section de mémoire (146) de l'endoscope (102).

10. Appareil de stérilisation (140) selon l'une quelconque des revendications 1 à 8, dans lequel la section de lecture (144) comprend un dispositif de communication qui inclut un cordon de communication, qui peut être connecté à la section de mémoire (146) de l'endoscope (102), et reçoit les informations provenant de la section de mémorisation d'informations par l'intermédiaire du cordon de communication.

11. Procédé de stérilisation, comprenant:
une étape d'exécution de traitement consistant à exécuter une étape de stérilisation consistant à stériliser un endoscope (102) en appliquant de la chaleur et de la vapeur sous haute pression, et une étape de séchage consistant à sécher l'endoscope (102) qui est stérilisé avec la vapeur;
une étape de lecture qui lit à partir d'une section de mémoire (146) de l'endoscope, des informations préenregistrées qui incluent une condition de traitement d'au moins l'une parmi l'étape de stérilisation et l'étape de séchage, et un type d'endoscope (102);
une étape de calcul qui calcule au moins une condition de traitement qui doit être exécutée actuellement, qui se rapporte à au moins l'une parmi l'étape de stérilisation et l'étape de séchage, sur la base des informations qui sont lues par l'étape de lecture; et
une étape de commande qui commande une opération de traitement d'au moins l'une parmi l'étape de stérilisation et l'étape de séchage, qui se rapporte à la condition de traitement, conformément à la condition de traitement qui est calculée à l'étape de calcul;
**caractérisé par**:
une étape de détection qui détecte la condition de traitement de ladite au moins l'une parmi l'étape de stérilisation et l'étape de séchage; et
une étape d'écriture qui écrit la condition de traitement détectée de ladite au moins l'une parmi l'étape de stérilisation et l'étape de séchage en tant qu'information historique dans la section de mémoire (146) de l'endoscope (102).

12. Procédé selon la revendication 11, dans lequel l'étape de calcul calcule une pluralité de conditions de traitement différentes qui doivent être exécutées actuellement,
comprenant en outre une étape d'indication qui indique ladite pluralité de conditions de traitement différentes qui sont calculées par l'étape de calcul; et
une étape de sélection destinée à sélectionner une condition de traitement qui doit être exécutée pour l'endoscope (102), à partir de la pluralité de conditions de traitement indicatives différentes qui sont indiquées par l'étape d'indication.

13. Procédé selon la revendication 12, dans lequel l'étape de calcul calcule une pluralité de conditions de traitement différentes incluant au moins deux temps d'étape, respectivement.

14. Procédé selon l'une quelconque des revendications 11 à 13, comprenant en outre une étape d'aspiration qui aspire un gaz interne de l'endoscope (102) et une étape d'injection qui injecte, dans l'endoscope après l'étape d'aspiration, un autre gaz différent du gaz dans l'endoscope (102).

15. Procédé selon l'une quelconque des revendications 11 à 14, dans lequel l'étape de lecture lit les informations historiques précédentes écrites dans la section de mémoire (146), si les informations historiques sont mémorisées dans la mémoire, et dans lequel l'étape de calcul calcule ladite au moins une condition de traitement sur la base des informations historiques précédentes en plus des informations qui sont lues par l'étape de lecture.
